# EUROPÄISCHE PATENTSCHRIFT

(11) **EP 0 521 398 B1**
(45) Veröffentlichungstag und Bekanntmachung des Hinweises auf die Patenterteilung: **28.10.1998**
(21) Anmeldenummer: 92110757.9
(22) Anmeldetag: 26.06.1992
(51) Int. Cl.: A23J 7/00

(54) **Phospholipidische Zusammensetzung**
Phospholipids container composition
Composition contenant des phospholipides

(30) Priorität: 05.07.1991 DE 4122300; 17.06.1992 DE 4219715
(43) Veröffentlichungstag der Anmeldung: 07.01.1993
(73) Patentinhaber: RHONE-POULENC RORER, 50829 Köln (DE)
(72) Erfinder: Losch, Rainer, Dr. Dipl.-Chem., W-5300 Bonn 1 (DE); Günther, Bernd-Rainer, Dr. Dipl.-Chem., W-5010 Bergheim 15 (DE); Hager, Jörg, Dipl.-Ing., W-5000 Köln 30 (DE)
(74) Vertreter: Fritzsche, Thomas M., Dr.

(56) Entgegenhaltungen:
- DE-A- 2 948 607
- PATENT ABSTRACTS OF JAPAN vol. 014, no. 439 19. September 1990
- PATENT ABSTRACTS OF JAPAN vol. 007, no. 285 20. Dezember 1983
- PATENT ABSTRACTS OF JAPAN vol. 008, no. 114 26. Mai 1984
- PATENT ABSTRACTS OF JAPAN vol. 012, no. 209 15. Juni 1988

## Beschreibung

Die vorliegende Erfindung betrifft ein Verfahren zur Herstellung einer phospholipidischen Zusammensetzung mit dem Merkmal des Oberbegriffs des Patentanspruchs 1.

Phospholipide kommen sowohl in tierischen als auch in pflanzlichen Materialien vor und müssen aus diesen isoliert werden. Hauptquellen hierfür sind Eier, Soja-Bohnen, Ölsaaten sowie Ölfrüchte, wie beispielsweise Kokosnuß-Kopra, Palmenkerne, Erdnüsse, Raps, Sonnenblumenkerne, Ölpalmen und Oliven.

Zur Isolierung der Phospholipide aus pflanzlichen Produkten wird das entsprechende Pflanzenöl, beispielsweise durch Einleiten von geringen Mengen Wasserdampf oder Wasser, entschleimt. Die dabei entstehende phospholipidische Zusammensetzung, auch Lecithinschlamm genannt, die in der Regel zwischen etwa 8 und 59 Gew.% Phospholipide aufweist, wird nach unterschiedlichen Verfahren getrocknet, so daß nach der Trocknung ein sogenanntes Rohlecithin anfällt.

Dieses Rohlecithin weist, abhängig von dem jeweils eingesetzten ursprünglichen Lecithinschlamm, eine unterschiedliche chemische Zusammensetzung auf. So besteht das wohl wichtigste Rohlecithin, das Sojalecithin, nach der Trocknung nach Weber E.J.; J.A. O.C.S. 58, 898 (1981) aus ca.:

| | | | |
|---|---|---|---|
| Triglyceride | 34,2% | Phosphatidylcholin | 19,1% |
| Diglyceride | 0,4% | Lysophosphatidylcholin | 0,7% |
| Freie Fettsäuren | 0,4% | Phosphatidylethanolamin | 8,6% |
| Sonst. Neutrallipide | 0,8% | Phosphatidylinositol | 8,8% |
| Glykolipide | 6,5% | Phosphatidsäure | 4,2% |
| Kohlenhydrate | 6,7% | N-Acylphosphatidylethanolamin | 1,0% |
| | | Sonstige | 8,6% |

Mittels acetonischer Entölung können die Neutrallipide, wie beispielsweise Triglyceride, Diglyceride o. dgl., aus dem Rohlecithin entfernt werden. Das entölte Rohlecithin, auch Reinlecithin genannt, ist ein festes, rieselfähiges Pulver, welches im Markt als Lecithingranulat erhältlich ist. Der Phosphatidylcholingehalt liegt im Reinlecithin zwischen etwa 25 Gew.% und 30 Gew.%.

Eine Anreicherung des Phosphatidylcholins kann aus Rohlecithin oder Reinlecithin nach bekannten Verfahren vorgenommen werden. Vorzugsweise arbeitet man hier nach den in den europäischen Patenten 00 54 770 oder 00 54 769 beschriebenen Verfahren. Hierbei wird das Rohlecithin mit Alkohol extrahiert. Die alkohollösliche Extraktionsphase wird über Siliciumdioxid bei erhöhten Temperaturen chromatografiert. Die daraus resultierende phospholipidische Zusammensetzung wird wegen des hohen Phosphatidylcholingehalts in Pharma-, Kosmetik- und Diäthetikbereichen eingesetzt. Sie weist jedoch den Nachteil auf, daß sie abhängig von ihrer chemischen Zusammensetzung pastenähnlich bzw. wachsartig ist. Die weitere Bearbeitung dieser Produkte bereitet dann häufig wegen ihrer Konsistenz erhebliche Schwierigkeiten, die sich in einer mangelnden Dosierbarkeit oder in einem Anhaften an Geräteinnenwandungen ausdrücken, so daß die in den Geräten verbleibenden Rückstände eine häufige und aufwendige Reinigung der Geräte erforderlich machen.

Um die zuvor beschriebenen Nachteile bei der Handhabung der hoch Phosphatidylcholin haltigen phospholipidischen Zusammensetzungen zu eliminieren, wurde bereits versucht, diese Produkte entsprechend weiterzubearbeiten, um so pulverförmige oder granulatähnliche hochreine Lecithine bzw. spezielle hochreine Phospholipide zu erhalten.

So schlägt beispielsweise die DE 973 741 C vor, Rohlecithin mit einem geeigneten Lösungsmittel, wie beispielsweise Aceton, zu entölen, anschließend das entölte Lecithin vom Ölextrakt abzutrennen, danach das abgetrennte Lecithin aufzulockern und in einer dünnen Schicht abzutrocknen. Hiernach kann das so entölte Lecithin von dem verwendeten Schichtträger flockenartig oder blättchenförmig abgetrennt werden. Ein derartiges Verfahren dürfte sich jedoch heute nicht mehr mit einem wirtschaftlich vertretbaren Aufwand realisieren lassen. Der Phosphatidylcholingehalt von etwa 30% ist des weiteren für die eingangs genannten Anwendungen zu niedrig.

Des weiteren sind Verfahren bekannt (US PS'en 20 57 695 und 30 12 888), bei denen Zusatzstoffe verwendet werden.

Ein ähnliches Verfahren wird in der DE 38 26 946 C vorgeschlagen. Hierbei wird während der Herstellung der hochreinen phospholipidischen Zusammensetzung als Zusatzstoff ein spezielles Zuckergemisch (Palatinit) eingesetzt, um so pulverartige oder granulierte hochreine spezielle Phospholipide bzw. hochreine phospholipidische Zusammensetzungen zu erhalten.

Die zuvor beschriebenen bekannten Verfahren weisen jedoch den Nachteil auf, daß hier ein zusätzlicher Aufwand, nämlich der Zusatz und die Einarbeitung des Zusatzstoffes, erforderlich ist. Darüber hinaus sind die nach den bekannten Verfahren hergestellten phospholipidischen Zusammensetzungen wegen der relativ geringen Phosphatidylcholinkonzentrationen nur sehr eingeschränkt verwendbar.

Um hochreine phospholipidische Zusammensetzungen herzustellen, ist es desweiteren bekannt, das entsprechende Lecithin in Wasser oder in einem Alkohol-Wasser-Gemisch zu fraktionieren. So beschreibt beispielsweise die JP 21 72 994 A ein Verfahren, bei dem eine wäßrige Suspension eines hochreinen Eilecithins hergestellt und anschließend lyophilisiert wird, wobei das so erstellte Produkt ein homogenes Pulver darstellen soll.

Das aus der DE 29 48 607 A bekannte Verfahren setzt zur Fraktionierung von Phospholipiden ein Alkohol-Wasser-Gemisch ein, wobei aus diesem Alkohol-Wasser-Gemisch eine Phosphatidylcholin-Fraktion ausgeflockt wird. Abhängig von dem eingesetzten Mischungsverhältnis von Wasser und Alkohol variiert bei dem bekannten Verfahren die Konzentration des Phosphatidylcholins in der Phospholipid-Ausflockung.

Die zuvor beschriebenen Verfahren weisen den Nachteil auf, daß zur Herstellung einer phosphatidylcholin-reichen Fraktion ein relativ großer Aufwand zu betreiben ist.

Der vorliegenden Erfindung liegt die Aufgabe zugrunde, ein Verfahren zur Herstellung einer phospholipidischen Zusammensetzung zur Verfügung zu stellen, das besonders einfach durchzuführen ist und das zu lagerstabilen Produkten führt.

Diese Aufgabe wird erfindungsgemäß durch ein Verfahren mit den kennzeichnenden Merkmalen des Patentanspruchs 1 gelöst.

Das erfindungsgemäße Verfahren zur Herstellung einer phospholipidischen Zusammensetzung, wobei die Zusammensetzung einen Phosphatidylcholin-Gehalt von mindestens 80 Gew.% aufweist und frei von Zusatzstoffen ist, sieht vor, daß man die hochreine phospholipidische Zusammensetzung mit einem Phosphatidylcholin-Gehalt von mindestens 80 Gew.% unter Kühlung bei einer Temperatur unterhalb von - 50 °C derart vermahlt, daß nach dem Vermahlen eine pulverisierte oder granulierte phospholipidische Zusammensetzung resultiert, deren Korn- bzw. Kristallitgröße zwischen 18 mm und 0,07 mm, vorzugsweise zwischen 6 mm und 0,5 mm, variiert.

Überraschend konnte festgestellt werden, daß sich phospholipidische Zusammensetzungen unterhalb der vorstehend genannten Temperatur problemlos vermahlen lassen, ohne daß es hierfür erforderlich ist, die bei den bekannten Verfahren eingesetzten Verfestigungsmittel zuor zuzumischen oder die zuvor erwähnte Fraktionierung durchzuführen. Auch blieben derartig zermahlene Zusammensetzungen, die einen Phosphatidylcholin-Gehalt von mindestens 80 Gew. % aufweisen, bis zu einer Temperatur von etwa 30° C über mehrere Monate und Jahre sogar stabil, so daß keine chemischen oder physikalischen Veränderungen auftreten.

Eine weitere, besonders bevorzugte Ausführungsform des erfindungsgemäßen Verfahrens sieht vor, daß man die phospholipidische Zusammensetzung vor der Vermahlung bei Temperaturen zwischen + 5° C und - 60° C, insbesondere bei Temperaturen zwischen - 10° C und - 30° C, lagert. Hierbei variiert abhängig von der jeweils ausgewählten Lagertemperatur die Verweilzeit zwischen einem Tag und 150 Tagen, vorzugsweise zwischen 30 Tagen und 70 Tagen. Eine derartig gelagerte phospholipidische Zusammensetzung kann dann innerhalb von kürzester Zeit auf die eingangs genannte Korngröße von 18 mm bis 0,07 mm, vorzugsweise von 6 mm bis 0,5 mm, vermahlt werden.

Abhängig von der jeweils eingesetzten phospholipidischen Zusammensetzung sieht eine weitere Ausführungsform des erfindungsgemäßen Verfahrens vor, daß man das Vermahlen und/oder die Lagerung der jeweiligen phospholipidischen Zusammensetzung unter Inertgas bei den zuvor aufgeführten Temperaturen und Lagerzeiten ausführt. Hier kommen insbesondere als Inertgase flüssiger Stickstoff, Kohlendioxid, ein Edelgas oder ein Gemisch der zuvor genannten Gase in Frage. Durch eine derartige Arbeitsweise unter Inertgas wird eine unerwünschte oxidative Veränderung der phospholipidischen Zusammensetzung verhindert.

Als Ausgangsmaterial können bei dem erfindungsgemäßen Verfahren die verschiedenen, aus Naturprodukten isolierten, hochreinen phospholipidischen Zusammensetzungen eingesetzt werden, sofern ihr Gehalt an Phosphatidylcholin mindestens 80 Gew % beträgt. Eine derartige Zusammensetzung kann vorzugsweise nach den bekannten Verfahren aus Eiern, Ölsaaten und Ölfrüchten und insbesondere aus Soja isoliert werden. Besonders geeignet ist es jedoch, wenn man bei dem erfindungsgemäßen Verfahren ein Sojalecithin einsetzt, dessen Gehalt an Phosphatidylcholin bei wenigstens 90 Gew. % und vorzugsweise bei 93 ± 3 Gew. % liegt. Ein derartiges hochreines Sojalecithin, das insbesondere zwischen 3 ± 3 gew% lysophosphatidylcholin enthält, kann beispielsweise nach den Verfahren isoliert werden, wie sie in den Europäischen Patentschriften 0 054 770 und 0 054 769 beschrieben sind.

Um das nach dem erfindungsgemäßen Verfahren hergestellte pulverisierte bzw. granulierte Produkt auch ohne chemische oder physikalische Veränderung über einen langen Zeitraum lagerstabil aufzubewahren, sieht eine weitere Ausführungsform des erfindungsgemäßen Verfahrens vor, daß es bei einer Temperatur kleiner als 10° C, vorzugsweise bei einer Temperatur zwischen 1° C und 6° C, gelagert wird.

Eine weitere Verbesserung der Lagerstabilität wird dann erreicht, wenn die vermahlene phospholipidische Zusammensetzung in luftdichten Beuteln, beispielsweise aus entsprechenden Aluminiumfolien, eingesiegelt und bei den zuvor genannten Temperaturen aufbewahrt wird.

Eine Weiterbildung des zuvor beschriebenen Verfahrens sieht dabei vor, daß nach dem Abfüllen der entsprechenden Beutel die darin enthaltene Luft noch durch ein Inertgas, beispielsweise Stickstoff, ein Edelgas, Kohlendioxid oder ein Gemisch der zuvor genannten Gase, verdrängt wird.

Wie bereits vorstehend beschrieben ist, kann die erfindungsgemäße pulverisierte bzw. granulierte phospholipidische Zusammensetzung sehr einfach gehandhabt und für verschiedene Zwecke eingesetzt werden. So ist es beispielsweise möglich, eine derartig pulverisierte bzw. granulierte phospholipidische Zusammensetzung als Lösungsvermittler, natürlichen Emulgator und/oder Liposomenbildner in kosmetischen Produkten und insbesondere in pharmazeutischen Produkten einzusetzen, da die erfindungsgemäße phospholipidische Zusammensetzung frei von Verfestigungsmitteln ist.

Vorteilhafte Weiterbildungen des erfindungsgemäßen Verfahrens sind in den Unteransprüchen angegeben.

Das erfindungsgemäße Verfahren wird nachfolgend anhand von Ausführungsbeispielen näher erläutert.

### Beispiel 1

300 Kg einer phospholipidischen Zusammensetzung mit einem Gehalt von 93% ± 3 Gew.% Phosphatidylcholin, die beispielsweise nach den in den EP PSen 00 54 770 oder 00 54 769 beschriebenen Verfahren aus Soja-Bohnen isoliert wurde, wurde über zwei Monate bei - 20° C gelagert. Unmittelbar vor der Vermahlung wurde die phospholipidische Zusammensetzung auf Raumtemperatur (17° C bis 21° C) gebracht. In einer üblichen Kaltmahlanlage wurde die Zusammensetzung dann unter Kühlung mit flüssigem Stickstoff vermahlen und unter Inertgas (Stickstoff) portionsweise in Aluminiumbeutel eingesiegelt. Das so verpackte Produkt wurde danach bei + 4° C gelagert.

Stabilitätstests ergaben, daß das Produkt bei einer Temperatur bis zu + 30° C seine Pulver- bzw. Granulatform beibehält.

Unmittelbar vor der Weiterverarbeitung eines derartigen Produktes wird dieses dann auf Raumtemperatur erwärmt, um so zu vermeiden, daß sich Kondensationsfeuchtigkeit auf dem vermahlten Produkt niederschlägt.

### Beispiel 2

Wie vorstehend beschrieben, wurden 100 Kg der zuvor angegebenen Zusammensetzung kaltvermahlen. Hierbei betrug jedoch abweichend vom Beispiel 1 die Lagerzeit vor der Vermahlung einen Monat bei - 17° C.

Stabilitätstests ergaben, daß selbst nach einer Lagerzeit nach dem Vermahlen und Abfüllen von 12 Wochen bei Zimmertemperatur (20° C ± 2° C) keine Änderung der Konsistenz auftrat.

Die so isolierte pulverisierte bzw. granulierte phospholipidische Zusammensetzung wies folgende chemische Zusammensetzung auf:

| | |
|---|---|
| Phosphatidylcholin | 94,2 Gew.% |
| Phosphatidylethanolamin | nicht nachweisbar |
| Phosphatidylinosit | nicht nachweisbar |
| Lyso-Phosphatidylcholin | 3,7 Gew.% |
| Wasser | 1,3 Gew. % |
| Triglyceride (Öle) | 0,8 Gew.% |

### Beispiel 3

Wie zuvor unter Beispiel 1 beschrieben, wurden 300 kg phospholipidische Zusammensetzung zusammen mit 150 kg Kohlendioxid-Granulat vermahlen. Hierbei entsprachen die übrigen Bedingungen den Bedingungen, wie sie in Beispiel 1 beschrieben sind.

Stabilitätstests ergaben, daß auch die nach dem Beispiel 3 hergestellte Zusammensetzung, die den zuvor angegebenen hohen Phosphatidylcholingehalt aufwies, selbst nach einer Lagerung nach dem Vermahlen von sechs Monaten unverändert in ihrer Konsistenz blieb.

## Patentansprüche

1. Verfahren zur Herstellung einer phospholipidischen Zusammensetzung mit einem Phosphatidylcholin-Gehalt von mindestens 80 Gew.%, wobei die phospholipidische Zusammensetzung frei von Zusatzstoffen ist, dadurch gekennzeichnet, daß man die hochreine phospholipidische Zusammensetzung mit einem Phosphatidylcholin-Gehalt von mindestens 80 Gew.% unter Kühlung bei einer Temperatur unterhalb von - 50 °C derart vermahlt, daß nach dem Vermahlen eine pulverisierte oder granulierte phospholipidische Zusammensetzung resultiert, deren Korn- bzw. Kristallitgröße zwischen 18 mm und 0,07 mm, vorzugsweise zwischen 6 mm und 0,5 mm, variiert.

2. Verfahren nach Anspruch 1, dadurch gekennzeichnet, daß man die phospholipidische Zusammensetzung vor der Vermahlung bei einer Temperatur zwischen + 5 °C und - 60 °C, vorzugsweise zwischen - 10 °C und - 30 °C, lagert.

3. Verfahren nach Anspruch 2, dadurch gekennzeichnet, daß man die phospholipidische Zusammensetzung zwischen einem Tag und 150 Tagen, vorzugsweise zwischen 30 Tagen und 70 Tagen, lagert.

4. Verfahren nach einem der vorangehenden Ansprüche, dadurch gekennzeichnet, daß man das Vermahlen und/oder die Lagerung der phospholipidischen Zusammensetzung unter Inertgas ausführt.

5. Verfahren nach Anspruch 4, dadurch gekennzeichnet, daß man als Inertgas Stickstoff, Kohlendioxid, ein Edelgas oder ein Gemisch dieser Gase auswählt.

6. Verfahren nach einem der vorangehenden Ansprüche, dadurch gekennzeichnet, daß die phospholipidische Zusammensetzung mindestens 90 Gew.% Phosphatidylcholin aufweist.

7. Verfahren nach Anspruch 6, dadurch gekennzeichnet, daß man eine phospholipidische Zusammensetzung auswählt, die aus Soja-Lecithin isoliert ist und die 93 ± 3 Gew.% Phosphatidylcholin enthält.

8. Verfahren nach Anspruch 7, dadurch gekennzeichnet, daß die phospholipidische Zusammensetzung 3 ± 3 Gew.% Lysophosphatidylcholin enthält.

9. Verfahren nach einem der Ansprüche 2 bis 4, dadurch gekennzeichnet, daß man nach der Lagerung und vor dem Vermahlen die Temperatur der phospholipidischen Zusammensetzung auf Raumtemperatur einstellt.

10. Verfahren nach einem der vorangehenden Ansprüche, dadurch gekennzeichnet, daß man die pulverisierte bzw. granulierte phospholipidische Zusammensetzung bei einer Temperatur kleiner als 10 °C, vorzugsweise bei einer Temperatur zwischen 1 °C und 6 °C, lagert.

11. Verfahren nach Anspruch 10, dadurch gekennzeichnet, daß man die pulverisierte bzw. granulierte phospholipidische Zusammensetzung unter Inertgas lagert.

12. Verfahren nach einem der vorangehenden Ansprüche, dadurch gekennzeichnet, daß die phospholipidische Zusammensetzung eine aus Soja-Lecithin isolierte Zusammensetzung ist.

## Claims

1. A method to manufacture a phospholipidic composition having a phosphatidylcholine content of at least 80 % by weight, whereby the phospholipidic composition is free from additives, characterised in that the highly pure phospholipidic composition having a phosphatidylcholine content of at least 80 % by weight is comminuted while being cooled at a temperature below - 50 °C in such a way that after the comminution a pulverized or granulated phospholipidic composition results which has a grain size, respectively a crystal size, varying between 18 mm and 0,07 mm, preferably between 6 mm and 0,5 mm.

2. The method according to claim 1, characterised in that the phospholipidic composition is stored at a temperature between + 5 °C and - 60 °C, preferably between - 10 °C and - 30 °C, before being comminuted.

3. The method according to claim 2, characterised in that the phospholipidic composition is stored during between one day and 150 days, preferably between 30 days and 70 days.

4. The method according to one of the preceding claims, characterised in that the comminution and/or the storing of the phospholipidic composition is carried out under an inert gas.

5. The method according to claim 4, characterised in that the inert gas is nitrogen, carbon dioxide, a noble gas or a mixture thereof.

6. The method according to one of the preceding claims, characterised in that the phospholipidic composition comprises at least 90 % by weight of phosphatidylcholine.

7. The method according to claim 6, characterised in that such a phospholipidic composition is chosen which is isolated from soy lecithin and which contains 93 ± 3 % by weight of phosphatidylcholine.

8. The method according to claim 7, characterised in that the phospholipidic composition contains 3 ± 3 % by weight of lysophosphatidylcholine.

9. The method according to one of the claims 2 to 4, characterised in that the temperature of the phospholipidic composition is set to room temperature after the storing and before the comminution step.

10. The method according to one of the preceding claims, characterised in that the pulverized, respectively granulated, phospholipidic composition is stored at a temperature of less than 10 °C, preferably at a temperature between 1 °C and 6 °C.

11. The method according to claim 10, characterised in that the pulverized, respectively granulated, phospholipidic composition is stored under inert gas.

12. The method according to one of the preceding claims, characterised in that the phospholipidic composition is isolated from soy lecithin.

## Revendications

1. Procédé de préparation d'une composition phospholipidique à teneur en phosphatidylcholine d'au moins 80% en poids, où la composition phospholipidique est dépourvue d'additifs, caractérisé en ce que l'on broie la composition phospholipidique de haute pureté, d'une teneur en phosphatidylcholine d'au moins 80% en poids, sous refroidissement et à une température inférieure à -50°C, en une manière telle qu'après le broyage, on obtienne une composition phospholipidique pulvérisée ou granulée, dont le calibre des grains ou des cristallites varie de 18 mm à 0,07 mm, de préférence, de 6 mm à 0,5 m.

2. Procédé suivant la revendication 1, caractérisé en ce que l'on conserve la composition phospholipidique avant le broyage à une température comprise entre +5°C et -60°C, de préférence, entre -10°C et -30°C.

3. Procédé suivant la revendication 2, caractérisé en ce que l'on conserve la composition phospholipidique entre un jour et 150 jours, de préférence, entre 30 jours et 70 jours.

4. Procédé suivant l'une quelconque des revendications précédentes, caractérisé en ce que l'on entreprend le broyage et/ou la conservation de la composition phospholipidique sous un gaz inerte.

5. Procédé suivant la revendication 4, caractérisé en ce que l'on choisit le gaz inerte parmi l'azote, le dioxyde de carbone, un gaz noble ou un mélange de ces gaz.

6. Procédé suivant l'une quelconque des revendications précédentes, caractérisé en ce que la composition phospholipidique présente au moins 90% en poids de phosphatidylcholine.

7. Procédé suivant la revendication 6, caractérisé en ce que l'on choisit une composition phospholipidique qui est isolée à partir de lécithine de soya et qui contient 93 ± 3% en poids de phosphatidylcholine.

8. Procédé suivant la revendications 7, caractérisé en ce que la composition phospholipidique contient 3 ± 3% en poids de lysophosphatidylcholine.

9. Procédé suivant l'une quelconque des revendications 2 à 4, caractérisé en ce qu'après la conservation et avant le broyage, on règle la température de la composition phospholipidique à la température ambiante.

10. Procédé suivant l'une quelconque des revendications précédentes, caractérisé en ce que l'on conserve la composition phospholipidique pulvérisée ou granulée à une température inférieure à 10°C, de préférence, une température comprise entre 1°C et 6°C.

11. Procédé suivant la revendication 10, caractérisé en ce que l'on conserve la composition phospholipidique pulvérisée ou granulée sous un gaz inerte.

12. Procédé suivant l'une quelconque des revendications précédentes, caractérisé en ce que la composition phospholipidique est une composition isolée de lécithine de soya.
